# EUROPEAN PATENT APPLICATION

(11) **EP 0 933 347 A1**
(43) Date of publication of application: **04.08.1999**
(21) Application number: 97122943.0
(22) Date of filing: 29.12.1997
(51) Int. Cl.: C07C 51/14, C07C 57/03, B01J 21/18, B01J 23/46, B01J 37/08

(54) **Process for the preparation of a carboxylic acid**

(71) Applicant: DSM N.V., 6411 TE Heerlen (NL)
(72) Inventor: Protzmann, Guido, 45772 Marl (DE); Luft, Gerhard, 64367 Mühltal (DE)

(57) **Abstract**

Process for the preparation of a carboxylic acid by carbonylating an alkenically unsaturated organic compound with carbon monoxide and water in the presence of an iodide or bromide promotor respectively and a supported rhodium catalyst, wherein the carbonylation is performed in the presence of an iodide or bromide salt respectively.

## Description

The invention relates to a process for the preparation of a carboxylic acid by carbonylating an alkenically unsaturated organic compound with carbon monoxide and water in the presence of an iodide or bromide promotor respectively and a supported rhodium catalyst.

Such a process is known from WO-A-9702091. Example IV discloses the carbonylation of butadiene to pentenoic acid with water in the presence of hydrogen iodide in the liquid phase and a supported catalyst comprising iodide, rhodium and an activated carbon support.

A drawback of this process is that the selectivity to pentenoic acid is only 91%. This is disadvantageous if the desired product is pentenoic acid.

The object of the present invention is to provide a process for the carbonylation of an alkenically unsaturated organic compound with a high selectivity with respect to the carboxylic acid.

This object is achieved in that the carbonylation is performed in the presence of an iodide or bromide salt respectively.

It has been found that in the process according to the invention less valerolactone, which is an undesired byproduct, is formed.

In case the process of the present invention is promoted with an iodide promotor respectively a bromide promotor, the process is performed in the presence of an iodide salt respectively a bromide salt.

Examples of the iodide or bromide salt present in the carbonylation process of the invention are quaternary ammonium iodides or bromides, phosphonium iodides or bromides and alkali metal iodides or bromides. Preferred iodide or bromide salts are alkali metal iodides or bromides. Examples of alkali metal iodides or bromides are lithium, sodium, potassium, cesium and rubidium iodide or bromide. Preferred alkali metal iodides or bromides are lithium, sodium and potassium iodide or bromide.

The molar ratio dissolved iodide or bromide salt and heterogeneous rhodium is preferably between 1:1 and 10:1 as calculated for a volume of reaction mixture and supported catalyst.

A dissolved iodide or bromide promotor has to be present in the reaction mixture in order to achieve a satisfactory reaction rate. Examples of suitable promotors are bromine, iodine, HI, HBr, organic bromide compounds, organic iodide compounds, and mixtures thereof. Preferred promoters are HI, HBr, acetyl bromide, acetyl iodide, lower alkyl bromides (C₁-C₁₀) and lower alkyl iodides (C₁-C₁₀), for example methyl bromide, bromoethane, 1-bromobutane, 1,4-dibromobutane, 2-bromopropane, 1-bromopropane, bromoheptane, methyl iodide, iodoethane, 1-iodobutane, 1,4-di-iodobutane, 2-iodopropane, 1-iodopropane and iodoheptane. The most preferred promotors are HI, HBR, methylbromide and methyliodide.

The molar ratio dissolved iodide or bromide and heterogeneous rhodium is preferably between 1:4 and 4:1 as calculated for a volume of reaction mixture and supported catalyst.

The process according to the invention is preferably performed in the presence of an iodide promotor and an iodide salt.

A more preferred embodiment of the process according to the present invention is a process in which an alkenically unsaturated organic compound is carboxylated with carbon monoxid and water in the presence of HI or methyliodide as the iodide promotor and an alkali metal iodide, preferably lithium, sodium or potassium iodide, as the iodide salt.

The process according to the invention is performed in the presence of a supported rhodium catalyst.

The support is for example activated carbon, clay, alumina, silica, silica-alumina, ceramics or a polymeric support. Examples of possible polymeric supports are polyvinylpyridine copolymers or styrene-divinyl-benzene copolymers containing diphenyl phosphine groups. The supported rhodium catalyst can be prepared by dispersing a rhodium source on the support. The rhodium source can be any material which will produce rhodium ions when contacting the rhodium source with the support. Among the materials which can be employed as the source of the rhodium are rhodium salts, oxides, rhodium carbonyl compounds and coordination compounds of rhodium. Examples of Rh-sources are RhI₃, RH(CO)₂I₃, Rh(III)nitrate trihydrate, Rh(CO)I₃, Rh₄(CO)₁₂, Rh₆(CO)₁₆, Rh(acac)₃, Rh(CO)₂(acac), Rh(C₂H₄)₂(acac), [Rh(C₂H₄)₂Cl]₂, [Rh(CO)₂Cl]₂, [Rh(COD)Cl]₂, Rh₂[O₂C(CH₂)₆CH₃]₄, Rh₂(acetate)₄, [Rh₂Cl₂(CO)₄] or RhCl₃.3H₂O, where acac is acetylacetonate and COD is 1,5-cyclooctadiene.

Preferably the supported rhodium catalyst comprises halogen atoms, rhodium and a support. The halogen atom can be for example F, Cl, Br, or I. Preferably X is Br or I and more preferably X is I. Preferably the support is an activated carbon support. The most preferably support is the activated carbon support disclosed in the above mentioned WO-A-9702091. This support has a more hydrophobic surface than normal activated carbon, obtainable by subjecting the activated carbon to a temperature treatment in an inert medium, in which the temperature is between 500 and 1100°C. The rhodium and the halogen is bound to the activated carbon support as shown in the following general formula: 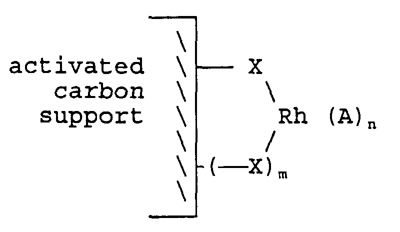 in which X represents the halogen atom, A an organic or anorganic group, n is 0-4 and m is 0-3.

'Alkenically unsaturated organic compounds' are understood to be compounds which, under the carbonylation conditions using the process according to the invention, are converted substantially into carboxylic acids. The carboxylic acid can subsequently be esterified with an alkanol to an alkyl carboxylate. The alkyl group of the alkylcarboxylate corresponds to the alcohol used. The alcohol is for example methanol, ethanol or propanol. The alkenically unsaturated organic compound can be represented by the following general formulae: 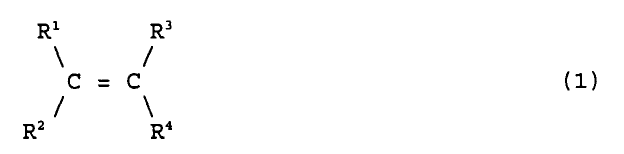 wherein R¹, R², R³ and R⁴ each independently represents hydrogen, halogen, alkyl, alkenyl, aryl, cycloalkyl, cycloalkenyl and an oxygenated hydrocarbyl group having from 0 to 20 carbon atoms.

Suitable alkenically unsaturated organic compounds are for example ethylene, propylene, butene-1, butene-2, isobutene, the hexenes, octenes, dodecenes, hexadecenes, 2-methylpropene, hexadiene, 1,3-butadiene, 2-methyl-1,3-butadiene, 2,3-dimethyl-1,3-butadiene, cyclohexene, methylcyclohexene, styrene, methylstyrene, vinylcyclohexene, 3,3-dimethyl-1-butene, 1,4-hexadiene, 2,4-hexadiene, 1,5-hexadiene, 2-methyl-1,4-hexadiene, acrolein, methyl vinyl ketone, 2-phenyl-2-butene, cyclopentadiene, 2-cyclohexyl-1-butene, 1,5-cyclooctadiene and 1,5,9-cyclododecatriene.

The alkenically unsaturated organic compound is preferably butadiene.

The temperature of the carbonylation reaction is generally between 40°C and 200°C.

The carbon monoxid partial pressure applied during the carbonylation reaction is in the range between 0.5 and 20 MPa.

A preferred embodiment of the process according to the invention is a process for the preparation of pentenoic acid in which butadiene is carbonylated in a suitable organic solvent with carbon monoxide and water in the presence of an alkali metal iodide, preferably lithium, sodium or potassium iodide, an iodide promotor and an on an activated carbon supported Rh/I catalyst. It has surprisingly been found that the selectivity to pentenoic acid is substantially improved compared with the process of WO-A-9702091. The resulting pentenoic acid is an important intermediate in a process to prepare adipic acid (precursor for Nylon-6.6) or ε-caprolactam (precursor for Nylon-6).

When the product is further subjected to a hydroformylation reaction as for example described in WO-A-9733854, the pentenoic acid is preferably first esterified with an alcohol, preferably methanol or ethanol, to the corresponding pentenoate.

The organic solvent which is used in this preferred embodiment of the invention is preferably acetic acid, the saturated carboxylic acids which are formed as by-products in the carbonylation and high boiling carboxylic acids (with a boiling point higher than that of pentenoic acid). For example adipic acid, valeric acid and/or C₉-carboxylic acids, alkyliodides, for example methyliodide, 2-iodobutane, 1-iodobutane, 2-iodobutene or 1-iodobutene.

Water should preferably not be present in a large excess. Preferably water is present in less than 15 wt% and more preferably in less than 10 wt% calculated on the total liquid reaction mixture including the solvent(s), promoter(s) and reactants but without the supported catalyst.

The process may be performed in for example a slurry reactor or a packed bed reactor. After the reaction the carboxylic acid can be isolated by for example extraction.

The invention shall be elucidated with the following non-limiting examples. The catalyst used in the following examples is prepared with the process as described in Example II of WO-A-9702091.

### Example I

100 gram of activated carbon (DESOREX ED 47 of the LURGI company) was treated with an aqeous NaOH (20%) solution at 90°C for two weeks. Subsequently the carbon was subjected to a Soxleth-extraction with water for one week. Subsequently the carbon was subjected to a Soxleth-extraction with 2N HCl for four weeks. Subsequently the carbon was subjected to a Soxleth-extraction with water for three weeks. The thus obtained carbon had less than 1 wt.% of SiO₂, Fe₂O₃ and Al₂O₃ impurities.

The carbon support obtained was subsequently dried and heated in an inert nitrogen atmosphere (0.3 MPa), in which the temperature was increased with 900°C per hour to 900°C. The temperature was kept at 900°C for 16 hours. After cooling the carbon was contacted with water for two weeks in which water mixture oxygen was bubbled through.

In a 100 ml autoclave 15 g of this carbon support was contacted with a solution of 50 ml acetic acid, 0.67 g of 56% HI (3.0 mmol) and 146 mg of [Rh₂(CO)₄Cl₂] (0.38 mmol) at a pressure of 0.3 MPa H₂ and 1.7 MPa CO and at a temperature of 130°C for 72 hours. The liquid solution was removed from the catalyst while the pressure was maintained at 0.5 MPa.

The autoclave, which contained about 15 g of the Rh supported catalyst, was filled with a solution of 40 g acetic acid, 1.44 g H₂O, 36.6 mg (56%) HI (0.16 mmol), 64.3 mg LiI (0.48 mmol), and 0.4 g of propionic acid (internal standard).

The autoclave was pressurized to 4.0 MPa with CO and heated to 130°C. Butadiene (3.2 g) was injected immediately and the pressure was increased to 7.0 MPa with additional CO. After 117, 261, 473 and 1400 minutes a sample was taken and analyzed by gas chromatography. Subsequently the pressure was let off to 0.5 MPa and the liquid solution was separated from the catalyst which remained in the autoclave. See table I for results.

**TABLE I**

| | | | | |
|---|---|---|---|---|
| Time (min) | 117 | 261 | 473 | 1400 |

| Concentration [mmol/kg reaction mixture] | | | | |
|---|---|---|---|---|
| 3-pentenoic acid | 106 | 243 | 431 | 532 |
| valerolactone | 1 | 3 | 5 | 17 |

### Example II

Example I was repeated, except that now 72 mg NaI (0.48 mmol) instead of 0.48 mmol LiI is used. Samples were taken after 92, 288, 416 and 1400 min. See table II for results.

**TABLE II**

| | | | | |
|---|---|---|---|---|
| Time (min) | 92 | 288 | 416 | 1400 |

| Concentration [mmol/kg reaction mixture] | | | | |
|---|---|---|---|---|
| 3-pentenoic acid | 93 | 272 | 373 | 541 |
| valerolactone | 2 | 3 | 5 | 18 |

### Example III

Example I was repeated, except that now 9 mg (56%) HI (0.04 mmol) and 90 mg NaI (0.60 mmol) instead of 0.16 mmol HI and 0.48 mmol LiI, is used. Samples were taken after 103, 304, 444 and 1400 min. See table III for results.

**TABLE III**

| | | | | |
|---|---|---|---|---|
| Time (min) | 130 | 304 | 444 | 1400 |

| Concentration [mmol/kg reaction mixture] | | | | |
|---|---|---|---|---|
| 3-pentenoic acid | 95 | 268 | 396 | 534 |
| valerolactone | 1 | 2 | 5 | 14 |

### Example IV

Example I was repeated, except that now 80 mg KI (0.48 mmol) is used instead of 0.48 mmol LiI. Samples were taken at 108, 281, 482 and 1400 min. See table IV for results.

**Table IV**

| | | | | |
|---|---|---|---|---|
| Time (min) | 108 | 281 | 482 | 1400 |

| Concentration [mmol/kg reaction mixture] | | | | |
|---|---|---|---|---|
| 3-pentenoic acid | 99 | 288 | 427 | 559 |
| valerolactone | 1 | 2 | 5 | 22 |

### Comparative Experiment A

Example I was repeated, except that now 145 mg (56%) HI (0.64 mmol) and no LiI is used, resulting in that the iodide (I⁻) concentration was the same as in Example I. Samples were taken at 92, 261, 441 and 1400 min. See table V for results.

**TABLE V**

| | | | | |
|---|---|---|---|---|
| Time (min) | 92 | 261 | 441 | 1400 |

| Concentration [mmol/kg reaction mixture] | | | | |
|---|---|---|---|---|
| 3-pentenoic acid | 128 | 264 | 380 | 427 |
| valerolactone | 3 | 6 | 11 | 75 |

## Claims

1. Process for the preparation of a carboxylic acid by carbonylating an alkenically unsaturated organic compound with carbon monoxide and water in the presence of an iodide or bromide promotor respectively and a supported rhodium catalyst, characterized in that the carbonylation is performed in the presence of an iodide or bromide salt respectively.

2. Process according to claim 1, characterized in that the iodide or bromide salt is an alkali metal iodide or alkali metal bromide.

3. Process according to any one of claims 1-2, characterized in that the molar ratio dissolved iodide or bromide salt and heterogeneous rhodium is between 1:1 and 10:1 as calculated for a volume of reaction mixture and supported catalyst.

4. Process according to any one of claims 1-3, characterized in that the carbonylation is performed in the presence of an iodide promotor and an iodide salt.

5. Process according to claim 6, characterized in that the iodide promotor is HI or methyliodide.

6. Process according to any one of claims 4-5, characterized in that the carbonylation is performed in the presence of an alkali metal iodide.

7. Process according to claim 6, characterized in that the alkali metal iodide is lithium iodide, sodium iodide or potassium iodide.

8. Process according to any one of claims 1-7, characterized in that the molar ratio dissolved iodide or bromide and heterogeneous rhodium is between 1:4 and 4:1 as calculated for a volume of reaction mixture and supported catalyst.

9. Process according to any one of claims 1-8, characterized in that the supported rhodium catalyst comprises halogen atoms, rhodium and an activated carbon support.

10. Process according to claim 9, characterized in that the support has a more hydrophobic surface than normal activated carbon, obtainable by subjecting the activated carbon to a temperature treatment in an inert medium, in which the temperature is between 500 and 1100°C.

11. Process according to any one of claims 9-10, characterized in that rhodium and halogen is bound to the activated carbon support as shown in the following general formula: 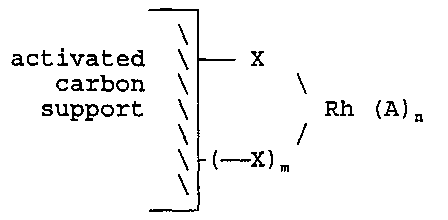 in which X represents the halogen atom, A an organic or anorganic group, n is 0-4 and m is 0-3.

12. Process according to any one of claims 10-11, characterized in that the halogen is iodine or bromide.

13. Process according to claim 12, characterized in that the halogen is iodine.

14. Process according to any one of claims 1-13, characterized in that the alkenically unsaturated organic compound is butadiene and the carboxylic acid is pentenoic acid.
